# EUROPEAN PATENT APPLICATION

(11) **EP 3 604 541 A1**
(43) Date of publication of application: **05.02.2020**
(21) Application number: 18778226.3
(22) Date of filing: 02.03.2018
(51) Int. Cl.: C12N 15/31, B01D 15/38, B01J 20/24, B01J 20/28, B01J 20/281, B01J 20/34, C07K 1/22, C07K 14/315, C07K 17/06, C07K 19/00, C12N 1/15, C12N 1/19, C12N 1/21, C12N 5/10, C12N 15/63

(54) **IMMUNOGLOBULIN-BINDING PEPTIDE WITH IMPROVED STABILITY**

(30) Priority: 31.03.2017 JP 2017071908
(71) Applicant: Kaneka Corporation, Osaka-shi, Osaka 530-8288 (JP)
(72) Inventor: YOSHIDA, Shinichi, Takasago-shi Hyogo 676-8688 (JP)
(74) Representative: Hoffmann Eitle
(86) International application number: PCT/JP2018/008050
(87) International publication number: WO 2018/180204

(57) **Abstract**

The objective of the present invention is to provide a novel altered Protein G which can bind to an immunoglobulin and which is excellent in a chemical stability to an alkaline solution, and an affinity separation matrix which has the altered Protein G as a ligand. The immunoglobulin-binding peptide according to the present invention is characterized in having the amino acid sequence designed by adding the specific mutation to the amino acid sequence cyclopaedically containing the amino acid residues common to antibody-binding domains derived from wild SpG.

## Description

### TECHNICAL FIELD

The present invention relates to an immunoglobulin-binding peptide of which chemical stability to an alkaline solution is improved, an affinity separation matrix which has the peptide as a ligand, a method for producing an antibody or an antibody fragment by using the affinity separation matrix, a DNA which encodes the peptide, a vector which contains the DNA, and a transformant which is transformed by the vector.

### BACKGROUND ART

One of the examples of important functions of a protein includes an ability to specifically bind to a specific molecule. The ability plays an important role in an immunoreaction and signal transduction in a living body. A technology utilizing the ability for purifying a useful substance has been actively developed. One of the examples of a protein which is actually utilized industrially includes Protein A affinity separation matrix. The Protein A affinity separation matrix is used for capturing an antibody drug to be purified with high purity at one time from a culture product of an animal cell (Non-patent documents 1 and 2). Hereinafter, Protein A is abbreviated as "SpA" in some cases. An antibody drug which has been developed is mainly a monoclonal antibody, and a monoclonal antibody has been produced on a large scale by using recombinant cell cultivation technology. A "monoclonal antibody" means an antibody obtained from a clone derived from a single antibody-producing cell. Most of antibody drugs launched presently are classified into an immunoglobulin G (IgG) subclass in terms of a molecular structure.

A protein referred to as Protein G found in Streptococcus sp. classified into Group G can also bind to IgG. Hereinafter, Protein G is referred to as "SpG" in some cases. An SpG affinity separation matrix product on which SpG is immobilized as a ligand has been commercially available (product name: "Protein-G Sepharose 4 Fast Flow" manufactured by GE Healthcare, Patent Document 1). SpG is characterized in strongly binding to an Fc region of IgG and also strongly binding to a broader kind of an animal IgG than SpA. It is further known that SpG weakly binds to a Fab region (Non-patent documents 3 and 4). The ability of SpG to bind to a Fab region is tried to be improved (Patent documents 2 to 4, Non-patent document 5).

An SpA affinity separation matrix is widely used for purifying an antibody drug in comparison with SpG. It is considered to be one of the reasons that SpA has higher stability to an alkaline solution than SpG (Non-patent document 1). When the stability to an alkali is high, such an affinity separation matrix can be regenerated to be repeatedly used by being washed with an aqueous solution of sodium hydroxide. A sodium hydroxide aqueous solution is inexpensive and highly effective in cleaning and sterilizing. The example of a method for enhancing the stability of a protein ligand of an affinity separation matrix to an alkali includes a protein engineering technique such as an introduction of an amino acid mutation. Specifically, a substitution of an asparagine residue or a glycine residue after an asparagine residue has an effect on the improvement of a chemical stability, since an asparagine residue is susceptible to deamination reaction under an alkaline condition (Non-patent document 1). On the one hand, all of such asparagine residue mutations in SpA do not necessarily show the improvement effect (Non-patent documents 1 and 6). A similar study to introduce a mutation in an asparagine residue is also made for SpG (Patent document 5, Non-patent documents 7 and 8); but there is a room for improvement, since not all mutations are effective as in the case of SpA and a stability to an alkali comparable to that of SpA has not been achieved.

### PRIOR ART DOCUMENT

### PATENT DOCUMENT

Patent Document 1: JP S63-503032 A
Patent Document 2: JP 2009-195184 A
Patent Document 3: WO 2015/030094
Patent Document 4: WO 2016/061427
Patent Document 5: JP 2016-079153 A

### NON-PATENT DOCUMENT

Non-patent Document 1: Hober S. et al., J. Chromatogr. B, 2007, vol. 848, pp. 40-47
Non-patent Document 2: Shukla A. A. et al., Trends Biotechnol., 2010, vol. 28, pp. 253-261
Non-patent Document 3: Bouvet P. J. et al., Int. J. Immunopharmac., 1994, vol. 16, pp. 419-424
Non-patent Document 4: Derrick J. P. et al., Nature, 1992, vol. 359, pp. 752-754
Non-patent Document 5: Bailey L. J. et al., J. Immunol. Methods, 2014, vol. 415, pp. 24-30
Non-patent Document 6: Linhult M. et al., PROTEINS, 2004, vol. 55, pp. 407-416
Non-patent Document 7: Gulich S. et al., Protein Eng., 2002, vol. 15, pp. 835-842
Non-patent Document 8 : Palmer B. et al., J. Biotechnol., 2008, vol. 134, pp. 222-230

### DISCLOSURE OF THE INVENTION

### PROBLEMS TO BE SOLVED BY THE INVENTION

The objective of the present invention is to provide a novel altered Protein G which can bind to an Fc region and a Fab region of an immunoglobulin and which is excellent in a chemical stability to an alkaline solution, an affinity separation matrix which has the altered Protein G as a ligand, and a method for producing an antibody or an antibody fragment by using the affinity separation matrix.

### MEANS FOR SOLVING THE PROBLEMS

The inventor of the present invention designed molecules of an altered IgG-binding domain of SpG, obtained the altered domains from a transformed cell by using a protein engineering method and a genetic engineering method, and compared properties of the obtained altered domains to solve the above-described problem.

Hereinafter, the present invention is described.
[1] An altered immunoglobulin-binding peptide selected from the following (1) to (3):
   (1) an immunoglobulin-binding peptide comprising an amino acid sequence of any one of SEQ ID NOs: 1 to 5 wherein an amino acid residue at the 8^{th} position is substituted by Asp, Glu, His, Ile, Lys, Leu or Val;
   (2) an immunoglobulin-binding peptide comprising an amino acid sequence specified in the (1) further having deletion, substitution and/or addition of one or more amino acid residues in a region except for the 8^{th} position;
   (3) an immunoglobulin-binding peptide comprising an amino acid sequence having a sequence identity of 80% or more with the amino acid sequence specified in the (1), provided that the amino acid substitution mutation specified in the (1) at the 8^{th} position is not further mutated in (3).
[2] The altered immunoglobulin-binding peptide according to the above [1], wherein the 8^{th} position of the amino acid sequence specified in the (1) is substituted by Ile, Leu or Lys.
[3] The altered immunoglobulin-binding peptide according to the above [1] or [2], wherein an amino acid position of the deletion, substitution and/or addition is one or more positions selected from the group essentially consisting of the 6^{th} position, the 7^{th} position, the 13^{th} position, the 15^{th} position, the 18^{th} position, the 19^{th} position, the 21^{st} position, the 24^{th} position, the 28^{th} position, the 29^{th} position, the 30^{th} position, the 31^{st} position, the 33^{rd} position, the 35^{th} position, the 39^{th} position, the 40^{th} position, the 42^{nd} position and the 47^{th} position in the amino acid sequence specified in the (2).
[4] The altered immunoglobulin-binding peptide according to any one of the above [1] to [3], wherein an amino acid position of the deletion, substitution and/or addition is N-terminal and/or C-terminal in the amino acid sequence specified in the (2).
[5] An altered immunoglobulin-binding peptide multimer comprising two or more of the altered immunoglobulin-binding peptides according to any one of the above [1] to [4] as domains connected one another.
[6] An affinity separation matrix comprising the altered immunoglobulin-binding peptide according to any one of the above [1] to [4] or the altered immunoglobulin-binding peptide multimer according to the above [5] and a water-insoluble carrier, wherein the altered immunoglobulin-binding peptide or the altered immunoglobulin-binding peptide is immobilized as a ligand on the water-insoluble carrier.
[7] A method for producing a protein comprising an Fc region and/or a Fab region of an immunoglobulin, comprising:
   contacting a liquid sample comprising the protein with the affinity separation matrix according to the above [6]; and
   separating the protein adsorbed on the affinity separation matrix from the affinity separation matrix.
[8] A DNA encoding the altered immunoglobulin-binding peptide according to any one of the above [1] to [4] or the altered immunoglobulin-binding peptide multimer according to the above [5].
[9] A vector comprising the DNA according to the above [8].
[10] A transformant transformed by the vector according to the above [9].

### EFFECT OF THE INVENTION

The immunoglobulin-binding activity of the chromatography carrier for affinity purification on which the altered SpG according to the present invention is immobilized is less likely to be decreased due to a damage by an alkaline treatment. It is therefore possible to wash the carrier by using a high concentration sodium hydroxide aqueous solution for a long time for repeated use.

The 8^{th} position of an IgG-binding domain of SpG corresponds to the 7^{th} position described in Non-patent document 7. When the position is substituted by Ala, an alkali resistance of such an altered SpG is hardly increased as Non-patent document 7. In addition, it is reported in Non-patent document 8 that altered SpGs of which the position is substituted by Gly and Thr exhibit high alkali resistance, but the alkali resistance by the present invention is superior to those of the altered SpGs.

Since many of the prior art documents are published as described-above, it is not easy to have an assumption that an alkali resistance can be remarkably improved by the other amino acid substitution. Though it is demonstrated by the present invention that the substitution by a branched chain amino acid, an acidic amino acid and a basic amino acid is effective, such a tendency is not general. The reason why such unpredictable effect can be obtained is considered to be that the conformational position, the involvement in binding to IgG and the sensitivity to an alkali of the 8^{th} position of the IgG-binding domain of SpG are complexly involved. The inventor also confirmed that such an effect cannot be obtained by an aromatic amino acid and a hydroxyamino acid. It can be said that a new methodology for improving the chemical stability to an alkali was found by the present invention beyond the scope of mere optimization.

### BRIEF DESCRIPTION OF THE DRAWINGS

Figure 1 is a graph prepared by plotting Fc-binding responses in each concentration of various GB1-N08X of Example 2 according to the present invention.
Figure 2 are graphs to demonstrate an alkali resistance of various GB1-N08X of Example 2 according to the present invention.

### MODE FOR CARRYING OUT THE INVENTION

The altered immunoglobulin-binding peptide according to the present invention is selected from the following (1) to (3) :
(1) an immunoglobulin-binding peptide comprising an amino acid sequence of any one of SEQ ID NOs: 1 to 5 wherein an amino acid residue at the 8^{th} position is substituted by Asp, Glu, His, Ile, Lys, Leu or Val;
(2) an immunoglobulin-binding peptide comprising an amino acid sequence specified in the (1) further having deletion, substitution and/or addition of one or more amino acid residues in a region except for the 8^{th} position;
(3) an immunoglobulin-binding peptide comprising an amino acid sequence having a sequence identity of 80% or more with the amino acid sequence specified in the (1), provided that the amino acid substitution mutation specified in the (1) at the 8^{th} position is not further mutated in (3).

An "immunoglobulin (IgG)" is a glycoprotein produced by a B cell of a lymphocyte and has a function to recognize a molecule such as a specific protein to be bound. An immunoglobulin has not only a function to specifically bind to a specific molecule referred to as antigen but also a function to detoxify and remove an antigen-containing factor in cooperation with other biological molecule or cell. An immunoglobulin is generally referred to as "antibody", and the name is inspired by such functions. All of immunoglobulins basically have the same molecular structure. The basic structure of an immunoglobulin is a Y-shaped four-chain structure consisting of two light chains and two heavy chains of polypeptides. A light chain (L chain) is classified into two types of λ chain and κ chain, and all of immunoglobulins have either of the types. A heavy chain (H chain) is classified into five types of γ chain, µ chain, α chain, δ chain and ε chain, and the kind of an immunoglobulin, i.e. isotype, is different depending on a heavy chain. An immunoglobulin G (IgG) is a monomer immunoglobulin, is composed of two heavy chains (γ chains) and two light chains, and has two antigen-binding sites.

A lower half vertical part in the "Y" shape of an immunoglobulin is referred to as an "Fc region", and an upper half "V" shaped part is referred to as a "Fab region". An Fc region has an effector function to initiate a reaction after an antibody binds to an antigen, and a Fab region has a function to bind to an antigen. The Fab region and Fc region of a heavy chain are bound to each other through a hinge part. Papain, which is a proteolytic enzyme and which is contained in papaya, decomposes a hinge part to cut into two Fab regions and one Fc region. The domain part close to the edge of the "Y" shape in a Fab region is referred to as a "variable region (V region)", since there are various changes in the amino acid sequence in order to bind to various antigens. A variable region of a light chain is referred to as a "VL region", and a variable region of a heavy chain is referred to as a "VH region". The other part in a Fab region except for a V region and an Fc region are referred to as a "constant region (C region)", since there is relatively less change. The constant region of a light chain is referred to as a "CL region", and the constant region of a heavy chain is referred to as a "CH region". A CH region is further classified into three regions of CH1 to CH3. A Fab region of a heavy chain is composed of a VH region and CH1, and an Fc region of a heavy chain is composed of CH2 and CH3. There is a hinge part between CH1 and CH2.

The term "peptide" in the present invention means any molecules having a polypeptide structure. In the range of the "peptide", not only a so-called protein but also a fragmented peptide and a peptide to which other peptide is bound through a peptide bond are included. The term "domain" means a unit of higher-order structure of a protein. A domain is composed of from dozens to hundreds of amino acid residues and means a protein unit which can sufficiently serve some kind of a physicochemical or biochemical function. The term "altered protein" and "altered peptide" mean a protein or peptide obtained by introducing at least one substitution, addition or deletion of an amino acid into a sequence of a wild protein or peptide. A mutation to substitute an amino acid is described by adding a wild or non-mutated amino acid residue before the number of substituted position and adding an amino acid residue for the mutation after the number of substituted position. For example, the mutation to substitute Asn at the 8^{th} position by Ala is described as N08A.

Protein G (SpG) is a protein derived from a cell wall of Streptococcus sp. classified into Group G. SpG has an ability to bind to an immunoglobulin G (IgG) of most mammals, strongly binds to an Fc region of IgG, and also weakly binds to a Fab region of IgG. SpG, in more detail, binds to an Fc region in the form of straddling CH2 and CH3 and a Fab region around a CH1 region (CH1γ), and a part of SpG also binds to a CL region (Non-patent documents 3 and 4). An altered SpG with enhanced binding ability to a Fab region has also been developed (Patent documents 2 to 4 and Non-patent document 5), and the present invention can be applied to such altered SpG.

An SpG functional domain having an IgG-binding ability is referred to as "β domain, i.e. SpG-β". The domain is referred to as β (B) domain or C domain (Akerstrom et al., J. Biol. Chem., 1987, 28, 13388-, Fig. 5), but is referred to as "β domain" in this disclosure in accordance with the definition of Fahnestock et al. (Fahnestock et al., J. Bacteriol., 1986, 167, 870-).

The immunoglobulin-binding peptide of the present invention binds to an Fc region and a Fab region of an immunoglobulin. The immunoglobulin to be bound by the present invention peptide is not particularly restricted as long as the immunoglobulin contains an Fc region or a Fab region, and may be an immunoglobulin which contains an Fc region and a Fab region without shortage or an IgG derivative. For example, the example of the IgG derivative includes a fragment which is fragmented into only an Fc region or a Fab region of an immunoglobulin G, a chimera IgG prepared by substituting a part of IgG domains with an IgG domain derived from other organism, an IgG of which sugar chain molecule of an Fc region is altered, an IgG which is covalently bound to a drug, and a fused peptide to which an Fc region or a Fab region is added as a tag.

The amino acid sequence of the above-described immunoglobulin-binding peptide (1) is basically an amino acid sequence which has a structure and a function of SpG-β. The present invention relates to a peptide having a sequence which corresponds to a sequence of an altered wild SpG-β. The example of the amino acid sequence before introducing the mutation includes sequences of wild SpG-β1 (SEQ ID NO: 1) and wild SpG-β2 (SEQ ID NO: 2). The N-terminal of wild SpG-β1 is Asp, but the N-terminal of SEQ ID NO: 1 is Thr as other domains. The N-terminal is not contained in the domain in accordance with a document such as Non-patent document 7, and the kind of amino acid at the N-terminal makes no difference. If the finally obtained peptide is included in the present invention range, an amino acid sequence except for the above-described sequence may be used. For example, it is clear that even the second domain from the N-terminal side of the three IgG-binding domains contained SpG derived from Group G streptococcus G148 strain or GX7805 strain exhibits similar effect. It is clear that the domain can be used in the present invention, since the domain has a sequence obtained by substituting Ile at the 6^{th} position with Val and Leu at the 7^{th} position with Ile in the amino acid sequence of wild SpGβ-1 (SEQ ID NO: 1) and shows an equivalent function. Many of altered Protein G excellent in thermal stability are known, and the example of such an amino acid sequence includes altered SpG-β2 (SEQ ID NO: 3) described in WO 97/26930 and JP 2003-88381 A. In addition, altered SpG-β which is described in Patent documents 2 to 4 and of which Fab-binding ability is improved is preferably used in the present invention, and for example, a further altered peptide of the altered SpG-β having the amino acid sequence of SEQ ID NO: 4 or SEQ ID NO: 5 is preferred as a Fab-binding peptide.

The altered immunoglobulin-binding peptide (1) of the present invention has the amino acid sequence of which above-described 8^{th} position is substituted. The 8^{th} position of the amino acid sequences of SEQ ID NOs: 1 to 5 is Asn.

The phrase, a peptide "has a (specific) amino acid sequence", in the present invention means that the specific amino acid sequence is contained in the amino acid sequence of the peptide and the function of the peptide is maintained. The sequence of a peptide other than a specific amino acid sequence in the peptide is exemplified by histidine tag, a linker sequence for immobilization, and a crosslinking structure such as -S-S- bond.

Even when, for example, a peptide is added to the N-terminal, a skilled person can easily identify the position corresponding to the 8^{th} position of SEQ ID NOs: 1 to 5 on the basis of the sequence identity. For example, the position can be identified by the alignment function of gene information processing software: GENETYX (https://www.genetyx.co.jp/).

The kind of an amino acid after mutation, specifically an amino acid which substitutes for Asn at the 8^{th} position of the amino acid sequences of SEQ ID NOs: 1 to 5, is not particularly restricted, the mutation may be a substitution by a non-protein-constituting amino acid and a non-natural amino acid, and a natural amino acid can be preferably used in terms of genetic engineering production. Asp, Glu, His, Ile, Lys, Leu or Val is preferred in terms of the improvement of an alkali resistance. Val, Leu or Lys is more preferred, and Lys is even more preferred. A natural amino acid is classified into the categories of a neutral amino acid; an acidic amino acid such as Asp and Glu; and a basic amino acid such as Lys, Arg and His. A neutral amino acid is classified into the categories of an aliphatic amino acid; an imino acid such as Pro; and an aromatic amino acid such as Phe, Tyr and Trp. An aliphatic amino acid is classified into the categories of a small amino acid such as Gly and Ala; a branched amino acid such as Val, Leu and Ile; a hydroxy amino acid such as Ser and Thr; a sulfur-containing amino acid such as Cys and Met; and an acid amide amino acid such as Asn and Gln. Since Tyr has a phenolic hydroxyl group, Tyr may be classified into not only an aromatic amino acid but also a hydroxy amino acid. From another viewpoint, a natural amino acid may also be classified into the categories of a nonpolar amino acid with high hydrophobicity, such as Gly, Ala, Val, Leu, Ile, Trp, Cys, Met, Pro and Phe; a neutral polar amino acid such as Asn, Gln, Ser, Thr and Tyr; an acidic polar amino acid such as Asp and Glu; and a basic polar amino acid such as Lys, Arg and His. The amino acid for the substitution in the present invention is preferably a branched amino acid, an acidic amino acid or a basic amino acid, and more preferably a branched amino acid or a basic amino acid, from the above-described aspects.

The altered immunoglobulin-binding peptide (2) of the present invention corresponds to an immunoglobulin-binding peptide comprising an amino acid sequence specified in the (1) having one or more mutations selected from deletion, substitution and addition of one or more amino acid residues in a region except for the 8^{th} position.

The range of "one or more" of the altered immunoglobulin-binding peptide (2) according to the present invention is not particularly restricted as long as the altered immunoglobulin-binding peptide with deletion or the like has a strong binding ability to an immunoglobulin. The above-described range of "one or more" is exemplified by 30 or less, preferably 20 or less, more preferably 10 or less, even more preferably 7 or less, even more preferably 5 or less, and particularly preferably 3 or less, 1 or 2, or 1.

The position of one or more mutations may be any positions except for the 8^{th} position. For example, the mutation position is preferably one or more positions selected from the essentially consisting of the 6^{th} position, the 7^{th} position, the 19^{th} position, the 24^{th} position, the 28^{th} position, the 29^{th} position, the 31^{st} position, the 35^{th} position, the 40^{th} position, the 42^{nd} position and the 47^{th} position of SEQ ID NOs: 1 to 5. The kinds of amino acids at the positions are different between SEQ ID NOs: 1 to 3. The mutation position is also preferably one or more positions selected from the essentially consisting of the 13^{th} position, the 15^{th} position, the 18^{th} position, the 19^{th} position, the 21^{st} position, the 28^{th} position, the 30^{th} position, the 33^{rd} position and the 39^{th} position of SEQ ID NOs: 1 to 5. The kinds of amino acids at the positions are different between SEQ ID NOs: 1, 4 and 5.

The position of the deletion, substitution and/or addition of the amino acid residue is exemplified by N-terminal and/or C-terminal. The terminal positions are particularly preferred as the position of the deletion and/or addition. As the embodiment in which an amino acid sequence is added, it is exemplified that an amino acid sequence containing Lys or Cys, which are useful for immobilizing the peptide on a matrix, is added to the C-terminal side.

Even when the number of amino acids is changed by the above-described deletion or addition, the position of an amino acid residue after introducing the mutation which position corresponds to the position of an amino acid residue before introducing the mutation can be easily identified by alignment analysis between the amino acid sequences before and after introducing the mutation. The means of such an alignment analysis is widely known by a person skilled in the art as the explanation with respect to the altered immunoglobulin-binding peptide (1).

The sequence identity specified in the altered immunoglobulin-binding peptide (3) of the present invention is preferably not less than 80% or not less than 85%, more preferably not less than 90%, not less than 95% or not less than 98%, and even more preferably not less than 99.5% or not less than 99.8%. The sequence identity can be evaluated by a program for amino acid sequence multiple alignment, such as Clustal (http://www.clustal.org/omega/), as the explanation with respect to the altered immunoglobulin-binding peptide (1).

The substituted amino acid residue at the 8^{th} position in the amino acid sequence specified in the above-described (1) is not further mutated in (3). Even when the numbers of amino acids in the amino acid sequences before and after the introduction of the mutation are different, a skilled person can easily identify a position corresponding to the 8^{th} position of SEQ ID NO: 1 under the condition that the sequence identity is 80% or more.

The present invention relates to a technology to create an altered peptide characterized in the improvement of a chemical stability to an alkaline aqueous solution by introducing a substitution mutation of an amino acid in comparison with that before the mutation is introduced. The altered immunoglobulin-binding peptide (1) is less damaged by an alkali even when treated with an alkaline aqueous solution, and an immunoglobulin binding performance thereof is maintained at high level, as confirmed in the examples described later. In addition, the altered immunoglobulin-binding peptides (2) and (3) is also excellent in a chemical stability. Specifically, it is preferred that a chemical stability of the altered immunoglobulin-binding peptides (2) and (3) to an alkaline aqueous solution is superior to that of an immunoglobulin-binding peptide having any one of the amino acid sequences of SEQ ID NOs: 1 to 5, and it is more preferred that the chemical stability is equivalent or superior to that of the altered immunoglobulin-binding peptide (1) having the mutation at the 8^{th} position of SEQ ID NOs: 1 to 5. In addition, it is preferred that a binding activity of the altered immunoglobulin-binding peptides (2) and (3) to an Fc region and/or a Fab region is superior to that of an immunoglobulin-binding peptide having any one of the amino acid sequences of SEQ ID NOs: 1 to 5, and it is more preferred that the binding activity is equivalent or superior to that of the altered immunoglobulin-binding peptide (1).

An "alkaline aqueous solution" shows an alkalinity to the extent that the purpose of cleaning or sterilization can be achieved. More specifically, a sodium hydroxide aqueous solution of 0.01 M or more and 1.0 M or less or 0.01 N or more and 1.0 N or less can be used, but the alkaline aqueous solution is not restricted thereto. With respect to sodium hydroxide as an example, the lower limit of the concentration thereof is preferably 10 mM, more preferably 15 mM, more preferably 20 mM, and even more preferably 25 mM. On the one hand, the upper limit of a sodium hydroxide concentration is preferably 1.0 M, more preferably 0.5 M, more preferably 0.3 M, more preferably 0.2 M, and even more preferably 0.1 M. The alkaline aqueous solution is not necessarily a sodium hydroxide aqueous solution. A pH thereof is preferably 12 or more and 14 or less. With respect to the lower limit of the pH, 12.0 or more is preferred, and 12.5 or more is more preferred. With respect to the upper limit of the pH, 14 or less is preferred, 13.5 or less is more preferred, and 13.0 or less is even more preferred.

The term "chemical stability" means a property to maintain a function of the peptide against a chemical modification such as a chemical change of an amino acid residue and a chemical denaturation such as a transition and a breakage of an amide bond. The function to be maintained of the peptide in the present invention means a binding activity to an immunoglobulin. The term "binding activity to an immunoglobulin" in the present invention means a ratio of the polypeptide which retains an affinity for an antibody or an antibody fragment without chemical denaturation. Thus, when the "chemical stability" is higher, a degree of decrease of a binding activity to an immunoglobulin is smaller even after an immersion treatment in an alkaline aqueous solution. The term "alkali resistance" in this disclosure has the same meaning as "chemical stability under an alkaline condition".

The time to immerse the peptide in an alkali is not particularly restricted, since a damage to the peptide is critically different depending on an alkaline concentration and a temperature during the immersion. For example, in the case where a concentration of sodium hydroxide is 15 mM and a temperature during the immersion is room temperature, the lower limit time for the immersion in an alkali is preferably 30 minutes, more preferably 1 hour, more preferably 2 hours, more preferably 4 hours, more preferably 10 hours, and more preferably 20 hours, but is not particularly restricted thereto.

A binding activity to an antibody or an antibody fragment can be evaluated by a biosensor such as Biacore system (GE Healthcare) using a surface plasmon resonance principle, but the means is not restricted thereto. With respect to a condition for measuring a binding activity, a binding signal at the time of binding to an antibody or a fragment thereof may be detected. With respect to a measuring condition, the temperature is preferably adjusted to a constant temperature of 20°C or higher and 40°C or lower. The pH for evaluating a binding condition is preferably adjusted to a neutral condition of 5 or more and 8 or less. The example of a neutral buffer component includes phosphate, tris and bis-tris, but is not restricted thereto. A sodium chloride concentration in a buffer is not particularly restricted and is preferably 0 M or more and 0.15 M or less.

For example, an association constant (K_{A}) and a dissociation constant (K_{D}) can be used as a binding parameter to demonstrate the binding to an antibody or an antibody fragment (Nagata et al., "Real-Time Analysis Experimental Method for Interaction Between Biological Substances", Springer-Verlag Tokyo, 1998, p. 41). An association constant between the present invention peptide and an antibody or an antibody fragment can be measured by immobilizing a human IgG or a fragment thereof on a sensor tip and adding each altered domain into a flow channel under a condition of the temperature of 25°C and pH 7.4 in Biacore system. An association constant (K_{A}) of the present invention protein to a human immunoglobulin is preferably 1 x 10⁵ (M⁻¹) or more, and more preferably 1 x 10⁶ (M⁻¹) or more, but is not restricted thereto, since an association constant is changed depending on a kind of an immunoglobulin and a domain number of an immunoglobulin-binding peptide.

When a residual binding activity after an alkaline treatment is evaluated, K_{A} and K_{D} are inappropriate as a binding parameter, since when a ratio of a molecule which can bind to an antibody or an antibody fragment is changed by an alkaline treatment but a binding ability of one peptide molecule to an antibody or an antibody fragment is not changed, K_{A} and K_{D} are not changed as a parameter. When a residual binding activity of the peptide is evaluated, for example, a binding signal value or a theoretical maximum binding capacity (Rₘₐₓ) is preferably used but the standard is not restricted thereto. The binding signal value is measured to an antibody or an antibody fragment of the same concentration immobilized on a sensor chip before and after the peptide is subjected to an alkaline treatment. A theoretical maximum binding capacity (Rₘₐₓ) is a binding parameter of which unit is a resonance unit (RU), which demonstrates a value of a binding response. For example, binding signal values to an antibody or an antibody fragment of the same concentration before and after a chip on which the peptide is immobilized is subjected to an alkaline treatment may be compared.

Since residual binding activities are compared before and after an alkaline treatment, a residual binding activity can be basically shown as a percentage calculated by dividing a binding activity after the alkaline treatment as a numerator by a binding activity before the alkaline treatment as a denominator. The value is not particularly restricted as long as the value is higher than that of a peptide into which the mutation of the present invention is not introduced and which is treated by an alkali in the same condition, and the percentage is preferably 10% or more, more preferably 20% or more, even more preferably 30% or more, even more preferably 40% or more, and even more preferably 50% or more.

It is important on this occasion that the sample to be compared is different only in the point that the mutation of the present invention is not introduced in the sample, the amino acid sequence is the same, and the conditions of an alkaline treatment and a measurement condition of a residual binding activity are the same. It is needed that the present invention peptide is appropriately treated, since the present invention peptide does not exhibit a binding activity to an immunoglobulin in an alkaline aqueous solution. For example, the pH after an alkaline treatment is adjusted to neutrality by an acid.

Protein G is a protein which contains 2 or 3 immunoglobulin-binding domains in the form of tandem line. As one of the embodiments, the altered immunoglobulin-binding peptide according to the present invention may be a multimer of 2 or more monomers or single domains of the altered immunoglobulin-binding peptide connected each other. The number of the monomers or single domains is preferably 3 or more, more preferably 4 or more, and even more preferably 5 or more. The upper limit of the number of connected domains may be, for example, 10, preferably 8, and more preferably 6. Such a multimer may be a homomultimer in which one kind of altered immunoglobulin-binding peptides are connected, such as homodimer and homotrimer, or a heteromultimer in which two or more kinds of altered immunoglobulin-binding peptides are connected, such as heterodimer and heterotrimer.

A method for connecting the altered immunoglobulin-binding peptides according to the present invention is exemplified by a connecting method through one or more amino acid residues and a method for directly connecting the monomer proteins without using an amino acid residue; however, is not restricted to the exemplified methods. The number of the amino acid residue for connection is not particularly restricted, and is preferably 1 residue or more and 20 residues or less, more preferably 15 residues or less, even more preferably 10 residues or less, even more preferably 5 residues or less, and even more preferably 2 residues or less. It is preferred to use a sequence which connects β1 and β2 or β2 and β3 of wild SpG. From another point of view, it is preferred that the amino acid residue for connection does not destabilize a three dimensional structure of the monomer altered immunoglobulin-binding peptide.

The altered immunoglobulin-binding peptide according to the present invention may contain any one of the amino acid sequences of the altered immunoglobulin-binding peptides (1) to (3), and other peptide and compound may be bound thereto. For example, a fusion peptide prepared by fusing the altered immunoglobulin-binding peptide according to the present invention or the peptide multimer of the two or more peptides as one structural component with other peptide having different function is exemplified as one embodiment. The example of such a fusion peptide includes a peptide fused with albumin or GST, i.e. glutathione S-transferase, but is not restricted to the examples. In addition, peptides fused with a nucleic acid such as DNA aptamer, a drug such as antibiotic or a polymer such as PEG, i.e. polyethylene glycol, are also included in the range of the present invention as long as such a fusion peptide utilizes the utility of the present invention peptide.

It is also included in the range of the present invention as one embodiment to use the above-described present invention peptide as an affinity ligand having an affinity for an immunoglobulin or a fragment thereof such as an Fc fragment and a Fab fragment. An affinity separation matrix prepared by immobilizing the ligand on a water-insoluble carrier is similarly included in the range of the present invention as one embodiment. The term "affinity ligand" in this disclosure means a substance and a functional group to selectively bind to and adsorb a target molecule from an aggregate of molecules on the basis of a specific affinity between molecules, such as binding between an antigen and an antibody, and means the peptide which specifically binds to an immunoglobulin or a fragment thereof in the present invention. The term "ligand" also means an "affinity ligand" in the present invention.

A water-insoluble carrier usable in the present invention is exemplified by an inorganic carrier such as glass beads and silica gel; an organic carrier; and a composite carrier obtained from the combination of the above carriers, such as an organic-organic composite carrier and an organic-inorganic composite carrier. The example of an organic carrier includes a carrier composed of synthetic polymer such as cross-linked polyvinyl alcohol, cross-linked polyacrylate, cross-linked polyacrylamide and cross-linked polystyrene; a carrier composed of polysaccharide such as crystalline cellulose, cross-linked cellulose, cross-linked agarose and cross-linked dextran. The commercial product thereof is exemplified by porous cellulose gel GCL2000, Sephacryl S-1000 prepared by crosslinking allyl dextran and methylene bisacrylamide through a covalent bond, an acrylate carrier Toyopearl, a cross-linked agarose carrier Sepharose CL4B, and a cross-linked cellulose carrier Cellufine. It should be noted that the water-insoluble carrier usable in the present invention is not restricted to the carriers exemplified as the above.

It is preferred that the water-insoluble carrier usable in the present invention has large surface area and that the carrier is porous with a large number of fine pores having a suitable size in terms of a purpose and method for using the affinity separation matrix according to the present invention. The carrier can have any form such as beads, monolith, fiber and film, and any form can be selected. A film may be a hollow fiber.

With respect to a method for immobilizing the ligand, for example, the ligand can be bound to a carrier by a conventional coupling method utilizing an amino group, a carboxy group or a thiol group of the ligand. Such a coupling method is exemplified by an immobilization method including activation of a carrier by a reaction with cyanogen bromide, epichlorohydrin, diglycidyl ether, tosyl chloride, tresyl chloride, hydrazine, sodium periodate or the like, or introduction of a reactive functional group on the carrier surface, and the coupling reaction between the resulting carrier and a compound to be immobilized as a ligand; and an immobilization method by condensation and crosslinking which method includes adding a condensation reagent such as carbodiimide or a reagent having a plurality of functional groups in the molecule, such as glutaraldehyde, into a mixture containing a carrier and a compound to be immobilized as a ligand.

A spacer molecule composed of a plurality of atoms may be introduced between the ligand and carrier. Alternatively, the ligand may be directly immobilized on the carrier. Accordingly, the altered immunoglobulin-binding peptide according to the present invention may be chemically modified for immobilization, or may have an amino acid residue useful for immobilization. Such an amino acid useful for immobilization is exemplified by an amino acid having a functional group useful for a chemical reaction for immobilization in a side chain, and specifically exemplified by Lys having an amino group in the side chain and Cys having a thiol group in the side chain. Since the immunoglobulin-binding ability of the peptide according to the present invention is principally maintained in a matrix prepared by immobilizing the peptide as a ligand in the present invention, any modification and change for immobilization are included in the range of the present invention.

It becomes possible by using the affinity separation matrix of the present invention that a protein containing an Fc region and/or a Fab region of an immunoglobulin is purified in accordance with affinity column chromatography purification method. A protein can be purified by a procedure in accordance with a method for purifying an immunoglobulin by affinity column chromatography (Non-Patent Document 1). Specifically, after a buffer which contains a protein and of which pH is approximately neutral is prepared, the solution is allowed to pass through an affinity column filled with the affinity separation matrix of the present invention so that the protein is selectively adsorbed. Then, an appropriate amount of a pure buffer is allowed to pass through the affinity column to wash the inside of the column. At the time, the target protein is still adsorbed on the affinity separation matrix of the present invention in the column. The affinity separation matrix on which the peptide according to the present invention is immobilized as a ligand is excellent in the absorption and retention performance of a target protein from the step to add a sample through the step to wash the matrix. Then, an acid buffer of which pH is appropriately adjusted is allowed to pass through the column to elute the target protein. As a result, purification with high purity can be achieved. Into the acid buffer for elution, a substance for promoting dissociation from the matrix may be added. The above-described target protein may be an immunoglobulin itself or an antibody fragment such as an Fc fragment and a Fab fragment.

The affinity separation matrix according to the present invention can be reused by allowing an adequate strong acidic or strong alkali pure buffer which does not completely impair the function of the ligand compound or the base material of the carrier to pass through the matrix for washing. An adequate modifying agent or an organic solvent may be contained in the buffer for regeneration. It is preferred to reuse the affinity separation matrix of the present invention by passing a pure strong alkaline buffer for washing, since the affinity separation matrix is particularly excellent in the chemical stability to an alkaline aqueous solution. The timing for regeneration by a pure strong alkaline buffer needs not to be every time after use and for example, may be once every five times or once every ten times.

The present invention also relates to a DNA which encodes the altered immunoglobulin-binding peptide of the present invention. The DNA encoding the present invention peptide may be any DNA as long as the amino acid sequence produced from translation of the base sequence of the DNA constitutes the present invention peptide. Such a base sequence can be obtained by a generally used known methods, for example, by polymerase chain reaction (hereinafter, abbreviated as "PCR") method. Alternatively, the DNA can be synthesized by publicly-known chemical synthesis method or is available from DNA library. A codon in the base sequence may be substituted by a degenerate codon, and the base sequence is not necessarily the same as the original base sequence as long as the translated amino acid is the same as that encoded by the original base sequence. It is possible to obtain a recombinant DNA having the one or more base sequences, a vector containing the recombinant DNA, such as a plasmid and a phage, a transformant transformed by the vector having the DNA, a genetically engineered organism having the DNA introduced therein, or a cell-free protein synthesis system using the DNA as a template for transcription.

The altered immunoglobulin-binding peptide according to the present invention may be obtained as a fusion peptide fused with a publicly-known protein which beneficially has an action to assist the expression of a protein or to facilitate the purification of a protein. In other words, it is possible to obtain a microorganism or cell containing at least one recombinant DNA encoding a fusion peptide containing the altered immunoglobulin-binding peptide according to the present invention. The example of the above-described protein includes a maltose-binding protein (MBP) and a glutathione S-transferase (GST), but is not restricted thereto.

Site-specific mutagenesis for modifying the DNA encoding the peptide of the present invention can be carried out by using recombinant DNA technology, PCR method or the like as follows. Specifically, mutagenesis by recombinant DNA technology can be carried out as follows: for example, in the case where there are suitable restriction enzyme recognition sequences on both sides of a target mutagenesis site in the gene encoding the present invention peptide, cassette mutagenesis method can be carried out in which method a desired site for mutagenesis is removed by cleaving the restriction enzyme recognition sites with the above-described restriction enzymes and then a mutated DNA fragment is inserted. In the mutated DNA fragment, mutation is introduced only at the target site by a method such as chemical synthesis.

For example, site-directed mutagenesis by PCR can be carried out by double primer mutagenesis. In double primer mutagenesis, PCR is carried out by using a double-stranded plasmid encoding the present invention peptide as a template, and using two kinds of synthesized oligo primers which contain complementary mutations in the + strand and - strand. A DNA encoding a multimer peptide can be produced by ligating the desired number of DNAs each encoding the monomer peptide (single domain) of the present invention to one another in tandem. For example, with respect to connecting method for the DNA encoding the multimer peptide, a suitable restriction enzyme site is introduced in the DNA sequence and double-stranded DNA fragments cleaved with the restriction enzyme are ligated by using a DNA ligase. One restriction enzyme site may be introduced or a plurality of restriction enzyme sites of different types may be introduced. When the base sequences encoding each monomer peptide in the DNA encoding the multimer peptide are the same, homologous recombination may be possibly induced in a host. Thus, the sequence identity between base sequences of DNAs encoding the ligated monomer peptides may be 90% or less, preferably 85% or less, more preferably 80% or less, and even more preferably 75% or less. The identity of the base sequence can be also determined by an ordinary method similarly to the amino acid sequence.

The "expression vector" of the present invention contains a base sequence encoding the above-described peptide of the present invention or a part of the amino acid sequence of the peptide, and a promoter which can be operably linked to the base sequence to function in a host. In general, the expression vector can be obtained by linking or inserting the gene encoding the present invention peptide to a suitable vector. The vector into which the gene is inserted is not particularly restricted as long as the vector is capable of autonomous replication in a host. A plasmid DNA or a phage DNA can be used as such a vector. For example, in the case where Escherichia coli is used as a host, a pQE series vector manufactured by QIAGEN, a pET series vector manufactured by Merck, a pGEX series vector manufactured by GE Healthcare Bioscience or the like can be used.

The transformant of the present invention can be produced by introducing the recombinant vector of the present invention into a host cell. A method for introducing the recombinant DNA into a host is exemplified by a method using a calcium ion, electroporation method, spheroplast method, lithium acetate method, agrobacterium infection method, particle gun method and polyethylene glycol method, but is not restricted thereto. A method for expressing the function of the obtained gene in a host is also exemplified by a method in which the gene obtained by the present invention is implanted into a genome (chromosome). A host cell is not particularly restricted, and bacteria (eubacteria) such as Escherichia coli, Bacillus subtilis, Brevibacillus, Staphylococcus, Streptococcus, Streptomyces and Corynebacterium can be preferably used in terms of mass production in a low cost.

The altered immunoglobulin-binding peptide according to the present invention can be produced by culturing the above-described transformant in a medium to allow the cell to produce and accumulate the present invention peptide in the cultured cell (including the periplasmic space of the cell) or in the culture liquid outside the cell, and collecting the desired peptide from the culture product. In addition, the peptide of the present invention can also be produced by culturing the above-described transformant in a medium to allow the cell to produce and accumulate a fusion protein containing the peptide of the present invention in the cultured cell (including the periplasmic space of the cell) or in the culture liquid outside the cell, collecting the fusion peptide from the culture product, cleaving the fusion peptide with a suitable protease, and collecting the desired peptide.

The transformant of the present invention can be cultured in a medium in accordance with a common method for culturing a host cell. The medium used for culturing the obtained transformant is not particularly restricted as long as the medium enables high yield production of the present invention peptide with high efficiency. Specifically, carbon source and nitrogen source, such as glucose, sucrose, glycerol, polypeptone, meat extract, yeast extract and casamino acid, can be used. In addition, an inorganic salt such as potassium salt, sodium salt, phosphate salt, magnesium salt, manganese salt, zinc salt and iron salt is added as required. In the case of an auxotrophic host cell, a nutritional substance necessary for the growth thereof may be added. In addition, an antibiotic such as penicillin, erythromycin, chloramphenicol and neomycin may be added as required.

Furthermore, in order to inhibit the degradation of the target peptide caused by a host-derived protease present inside or outside the transformant, a publicly-known protease inhibitor and/or other commercially available protease inhibitor may be added in an appropriate concentration. The publicly-known protease inhibitor is exemplified by phenylmethane sulfonyl fluoride (PMSF), benzamidine, 4-(2-aminoethyl)-benzenesulfonyl fluoride (AEBSF), antipain, chymostatin, leupeptin, Pepstatin A, phosphoramidon, aprotinin and ethylenediaminetetraacetic acid (EDTA).

In order to obtain rightly folded altered immunoglobulin-binding peptide according to the present invention, for example, a molecular chaperone such as GroEL/ES, Hsp70/DnaK, Hsp90 and Hsp104/ClpB may be used. For example, such a molecular chaperone is co-existed with the present invention peptide by coexpression or as a fusion protein. As a method for obtaining rightly folded present invention peptide, addition of an additive for assisting right folding into the medium and culturing at a low temperature are also exemplified, but the method is not restricted thereto.

The medium for culturing transformant produced from an Escherichia coli as a host is exemplified by LB medium containing triptone 1%, yeast extract 0.5% and NaCl 1%, 2xYT medium containing triptone 1.6%, yeast extract 1.0% and NaCl 0.5%, or the like. For example, the transformant may be aerobically cultured in an aeration-stirring condition at a temperature of 15 to 42°C, preferably 20 to 37°C, for several hours to several days. As a result, the peptide of the present invention is accumulated in the cultured cell (including the periplasmic space of the cell) or in the culture liquid outside the cell to recover the peptide. In some cases, the culturing may be performed anaerobically without aeration. In the case where a recombinant peptide is secreted, the produced recombinant peptide can be recovered after the culturing by separating the supernatant containing the secreted peptide by using a common separation method such as centrifugation and filtration from the cultured cell. In addition, in the case where the peptide is accumulated in the cultured cell (including the periplasmic space), the peptide accumulated in the cell can be recovered, for example, by collecting the cell from the culture liquid by centrifugation, filtration or the like, and then disrupting the cell by sonication, French press method or the like, and/or solubilizing the cell by adding a surfactant or the like.

A method for purifying the peptide of the present invention can be carried out by any one or an appropriate combination of techniques such as affinity chromatography, cation or anion exchange chromatography and gel filtration chromatography. It can be confirmed whether the obtained purified substance is the target peptide or not by an ordinary method such as SDS polyacrylamide gel electrophoresis, N-terminal amino acid sequence analysis and Western blot analysis.

The present application claims the benefit of the priority date of Japanese patent application No. 2017-71908 filed on March 31, 2017. All of the contents of the Japanese patent application No. 2017-71908 filed on March 31, 2017, are incorporated by reference herein.

### EXAMPLES

Hereinafter, the present invention is described in more detail with Examples. The present invention is however not restricted to the following Examples.

The altered peptide obtained in the following Examples is described as "domain - introduced mutation", and a peptide before a substitution mutation is introduced is described as "domain - Cont". For example, wild SpG-β1 having the amino acid sequence of SEQ ID NO: 1 is described as "GB1-Cont", and altered SpG-β1 into which a mutation of N08A to substitute Asn at the 8^{th} position of the amino acid sequence of SEQ ID NO: 1 by Ala is described as "GB1-N08A". Concurrently introduced mutations are written together by using a slash. For example, altered SpG-β1 into which mutations of N08A and D36V are introduced is described as "GB1-N08A/D36V". Various altered peptide of which 8^{th} position is mutated is collectively described as "N08X". Hereinafter, "wild SpG-β1" is simply referred to as "SpG-β1" in some cases.

With respect to a peptide in which a plurality of single domains are connected, the number of the connected domains and "d" are added together after a period. For example, a peptide in which two altered SpG-β1 domains having a mutation of N08A are connected is described as "GB1-N08A.2d". When a Cys residue, i.e. "C", having a functional group for immobilization is added to the C-terminal in order to immobilize a peptide on a water-insoluble carrier, one letter code of the added amino acid is added after "d". For example, an altered SpG-β1 domain in which two SpG-β1 domains which has a mutation of N08A are connected and to which Cys is added at the C-terminal is described as "GB1-N08A.2dC".

### Example 1: Preparation of GB1-N08X

### (1) Preparation of expression plasmid of GB1-N08X

The DNA sequences encoding the peptides having the amino acid sequences of various GB1-N08X were designed by reverse translation from the amino acid sequences. PCR was carried out by using three kinds of single strand oligo DNA (f31/f32/f33), and the PCR product was digested by using restriction enzymes BamHI/EcoRI to be integrated in the multi cloning site of an expression vector pGEX-6P-1 treated by the same restriction enzymes. In the PCR to prepare the encoding DNA, a small amount of f32 (0.2 µM, leading) and f33 (10 µM, lagging) were firstly extended by overlap extension PCR, and then double-stranded DNA formed by overlap extension PCR using f31 (10 µM, leading) and f33 (lagging) extended by the former reaction became an encoding DNA sequence having restriction enzyme sites at both ends. Overlap extension PCR using f31 and f32 was starting point in some cases, but the final products were the same. Various oligo DNA sequences corresponding to f31 to f33 of each GB1-N08X were designed to proceed the reaction, and the synthesis thereof was outsourced to Eurofins. With respect to the specific experimental procedure, PCR was carried out by using BlendTaq Plus (manufactured by TOYOBO) as a polymerase. The PCR product was subjected to agarose electrophoresis and the target band was cut out to extract double-stranded DNA. The double-stranded DNA was cleaved with the restriction enzymes BamHI and Eco52I (manufactured by Takara Bio). Then, a plasmid vector pGEX-6P-1 (GE Healthcare Bioscience) was similarly treated by BamHI/EcoRI, and then subjected to dephosphorylation treatment by a dephosphorylation enzyme CIAP (manufactured by Takara Bio) and ligation reaction using Ligation high (manufactured by TOYOBO).

The DNA encoding altered GB1-N08X was prepared by using oligo DNAs which consisted of f32 (SEQ ID NO: 6) and f33 (SEQ ID NO: 7) in common and f31 having different sequence. The sequence of oligo DNA of f31, f32 and f33, encoding DNA sequence, and SEQ ID NO of the amino acid sequence of each altered peptide are collectively described in Table 1.

**Table 1**

| Altered peptide | Oligo DNA | | | Encoding DNA | Amino acid |
|---|---|---|---|---|---|
| | f31 | f32 | f33 | | |
| GB1-N08D.1d | 8 | 6 | 7 | 15 | 22 |
| GB1-N08E.1d | 9 | 6 | 7 | 16 | 23 |
| GB1-N08H.1d | 10 | 6 | 7 | 17 | 24 |
| GB1-N08I.1d | 11 | 6 | 7 | 18 | 25 |
| GB1-N08K.1d | 12 | 6 | 7 | 19 | 26 |
| GB1-N08L.1d | 13 | 6 | 7 | 20 | 27 |
| GB1-N08V.1d | 14 | 6 | 7 | 21 | 28 |
| GB1#-N08K.1d | 29 | 30 | 7 | 31 | 32 |

Altered GB1#-N08K was similarly prepared. The altered GB1#-N08K corresponded to GB1#-Cont (SEQ ID NO: 4) which had strong binding ability to Fab and into which the mutation of N08K was introduced. The altered peptide was similarly prepared to the above-described method by using single strand oligo DNAs f31 (SEQ ID NO: 29), f32 (SEQ ID NO: 30) and f33 (SEQ ID NO: 7). The encoding DNA sequence is SEQ ID NO: 31 and the amino acid sequence is SEQ ID NO: 32 as Table 1.

A competent cell ("Escherichia coli HB101" manufactured by Takara Bio, Inc.) was transformed by using the above-described plasmid vector pGEX-6P-1 in accordance with the protocol attached to the competent cell product. By using the plasmid vector pGEX-6P-1, GB1-N08X.1d which was fused with glutathione-S-transferase (hereinafter, abbreviated as "GST") could be produced. Then, the plasmid DNA was amplified and extracted by using a plasmid purification kit ("Wizard Plus SV Minipreps DNA Purification System" manufactured by Promega) in accordance with the standard protocol attached to the kit. The base sequence of the encoding DNA of the expression plasmid was determined by using a DNA sequencer ("3130xl Genetic Analyzer" manufactured by Applied Biosystems). The sequencing PCR was performed by using a gene analysis kit ("BigDye Terminator v. 1.1 Cycle Sequencing Kit" manufactured by Applied Biosystems) and DNA primers for sequencing the plasmid vector pGEX-6P-1 (manufactured by GE Healthcare Bioscience) in accordance with the attached protocol. The sequencing product was purified by using a plasmid purification kit ("BigDye XTerminator Purification Kit" manufactured by Applied Biosystems) in accordance with the attached protocol and used for the base sequence analysis.

### (2) Preparation of GB1-N08X

The transformant cell produced by integrating each of the GB1-N08X gene or GB1#-N08X gene obtained in the above-described (1) was cultivated in 2xYT medium containing ampicillin at 37°C overnight. The culture liquid was inoculated in 2xYT medium containing about a 100-fold amount of ampicillin and cultivated at 37°C for about 1 hour and then at 25°C for about 1 hour. Then, isopropyl-1-thio-β-D-galactoside (IPTG) was added so that the final concentration thereof became 0.1 mM, and the transformant was further cultivated at 25°C for about 18 hours.

After the cultivation, the cell was collected by centrifugation and re-suspended in 5 mL of PBS buffer. The cell was broken by sonication and centrifuged to separate a supernatant fraction as a cell-free extract and an insoluble fraction. When a target gene is integrated into the multiple cloning site of pGEX-6P-1 vector, a fusion peptide having GST added to the N-terminal is produced. Each fraction was analyzed by SDS electrophoresis; as a result, a peptide band assumed to be induced by IPTG was detected at a position corresponding to a molecular weight of about 25,000 or more in the lanes of each of the cell-free extracts obtained from all of culture liquids of each transformant. The molecular weights were approximately similar but the positions of bands were different depending on the kind of altered peptide.

The GST fusion peptide was roughly purified from each of the cell-free extract containing the GST fusion peptide by affinity chromatography using a GSTrap FF column (GE Healthcare Bioscience), which had an affinity for GST. Each of the cell-free extract was added to the GSTrap FF column and the column was washed with a standard buffer (20 mM NaH₂PO₄-Na₂HPO₄, 150 mM NaCl, pH 7.4). Then, the target GST fusion peptide was eluted by using an elution buffer (50 mM Tris-HCl, 20 mM glutathione, pH 8.0). As the sample used for assay in the following Examples with fusing GST, a peptide solution obtained by concentrating the eluent and replacing the elution buffer with the standard buffer by using centrifugal filter unit Amicon (manufactured by Merck Millipore) was used.

When a gene is integrated into a multiple cloning site of pGEX-6P-1 vector, an amino acid sequence by which GST can be removed by using sequence-specific protease, "PreScission Protease" (manufactured by GE Healthcare Bioscience), is inserted between GST and a target peptide. By using such PreScission Protease, GST was removed in accordance with the attached protocol. The target peptide was purified by gel filtration chromatography using Superdex 75 10/300 GL column (manufactured by GE Healthcare Bioscience) from the GST-removed sample. Each of the reaction mixture was added to the Superdex 75 10/300 GL column equilibrated with the standard buffer, and the target peptide was separated and purified from the removed GST and PreScission Protease. The above-described all of the peptide purification by chromatography using the column was performed by using AKTAprime plus system (manufactured by GE Healthcare Bioscience). In addition, after the removal of GST, the peptide produced in the present Example had the sequence of Gly-Pro-Leu-Gly-Ser derived from the vector pGEX-6P-1 at the N-terminal side.

### Example 2: Evaluation of alkali resistance of GB1-N08X

### (1) Preparation of Fab fragment/Fc fragment

A humanized monoclonal IgG product was fragmented into a Fab fragment and an Fc fragment by using papain, and the Fc fragment and Fab fragment were purified. Hereinafter, each fragment is simply abbreviated as "Fc" and "Fab". Specifically, an anti-human TNFα monoclonal IgG product (general name: Infliximab, product name: Remicade, manufactured by Mitsubishi Tanabe Pharma Corporation) was dissolved in a buffer for digestion by papain (0.1 M AcOH-AcONa, 2 mM EDTA, 1 mM cysteine, pH 5.5), and agarose on which papain was immobilized ("Papain Agarose from papaya latex" manufactured by SIGMA) was added thereto. The mixture was incubated with stirring by a rotator at 37°C for about 8 hours. The Fab was purified from the Fc by recovering the Fab as a flow-through fraction in an affinity chromatography using KanCapA column (manufactured by KANEKA Corporation) from the reaction mixture which contained both of the Fab and Fc and which was separated from the agarose on which papain was immobilized. The Fc adsorbed on the column was eluted by 0.05 M acetic acid/sodium acetate buffer (pH 3.5) and immediately neutralized by 0.1 M Tris-HCl buffer (pH 8.0). The Fab solution and Fc solution were subjected to purification by gel filtration chromatography using Superdex 75 10/300 GL column (manufactured by GE Healthcare) to obtain a Fab solution and an Fc solution. In the chromatography, the standard buffer was used for equilibration and separation. Similarly to the above-described Example 1, AKTAprime plus system was used in the chromatography for protein purification.

### (2) Alkali treatment of GB1-N08X

Each GB1-N08X was subjected to dialysis using ultrapure water and the concentration thereof was adjusted to obtain 20 µM aqueous solution. To 0.2 mL of the aqueous solution, 0.1 mL corresponding to a half amount of the aqueous solution of 45 mM sodium hydroxide aqueous was added. The mixture was incubated at 25°C for 2 hours and then neutralized by 0.1 mL of 50 mM acetic acid (pH 3.0). After it was confirmed that the mixture was neutralized by using a pH test paper, the mixture was diluted to 5 times by the standard buffer.

### (3) Measurement of binding response of GB1-N08X to immunoglobulin derivative

The binding response of each of the GB1-N08X obtained in the above-described Example 1(2) to Fc was evaluated by using a biosensor Biacore 3000 (manufactured by GE Healthcare Bioscience) utilizing surface plasmon resonance. In the present Example, the Fc obtained in the above-described Example 2(1) was immobilized on a sensor tip, and each of the altered GB1-N08X was flown on the tip to detect the interaction between the two. The Fc was immobilized on a sensor tip CM5 by amine coupling method using N-hydroxysuccinimide (NHS) and N-ethyl-N'-(3-dimethylaminopropyl)carbodiimide hydrochloride (EDC), and ethanolamine was used for blocking. All of the sensor tip and reagents for immobilization were manufactured by GE Healthcare Bioscience. The Fc solution was diluted to about 10 times by using a buffer for immobilization (10 mM CH₃COOH-CH₃COONa, pH 4.5), and the Fc was immobilized on the sensor tip in accordance with the protocol attached to the Biacore 3000. In addition, a reference cell as negative control was also prepared by activating another flow cell on a tip with EDC/NHS and then immobilizing ethanolamine only. The immobilized amount of Fc was about 5000 RU. A detection sensitivity and a dependence degree on an analyte concentration were improved by adjusting the immobilization amount to high as 5000 RU or more and the flow rate to slow. In other words, a dependence degree of a binding response on an affinity was decreased and a dependence degree on a concentration was relatively increased under mass transport limited environment.

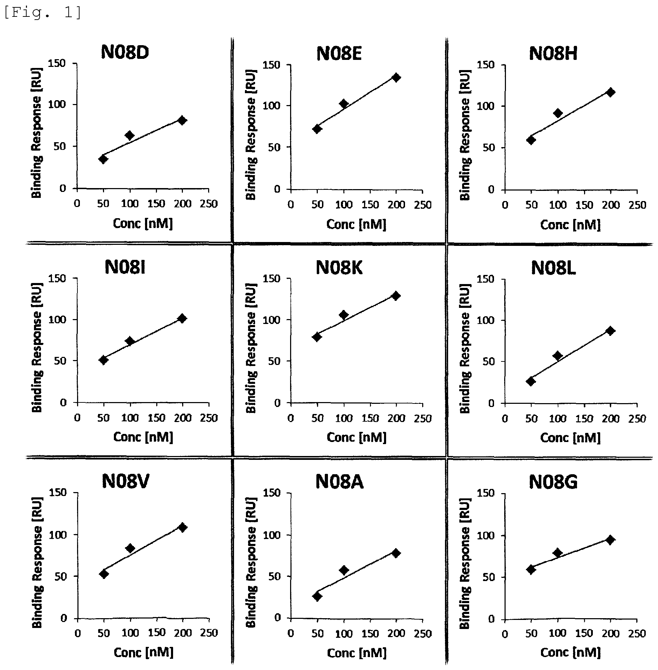

A protein solution having a concentration of 50 nM, 100 nM or 200 nM of GB1-N08X before the alkali treatment was prepared by using a running buffer (20 mM NaH₂PO₄-Na₂HPO₄, 150 mM NaCl, 0.005% P-20, pH 7.4). The protein solution was added to the sensor tip in a flow rate of 10 µL/min for 2 minutes. Bonding response curves at the time of addition (association phase, for 2 minutes) and after the addition (dissociation phase, for 2 minutes) were sequentially obtained at a measurement temperature of 25°C. After the measurement, washing was carried out by using about 20 mM NaOH. A graph prepared by plotting a binding response 1 minute after the addition, i.e. resonance unit value of the bonding response curve, on the vertical axis and the concentration of the added analyte at the time on the horizontal axis is shown as Figure 1. The binding response was somewhat proportional to the analyte concentration in the concentration range in this evaluation system. As Figure 1, the slope of the binding response to the analyte concentration is different depending on the altered peptide. In this evaluation system, the ratio of the responses before and after the alkali treatment was not merely compared but the correction to convert to concentration was performed to calculate a residual binding activity.

### (4) Residual Fc-binding activity of GB1-N08X after alkali treatment

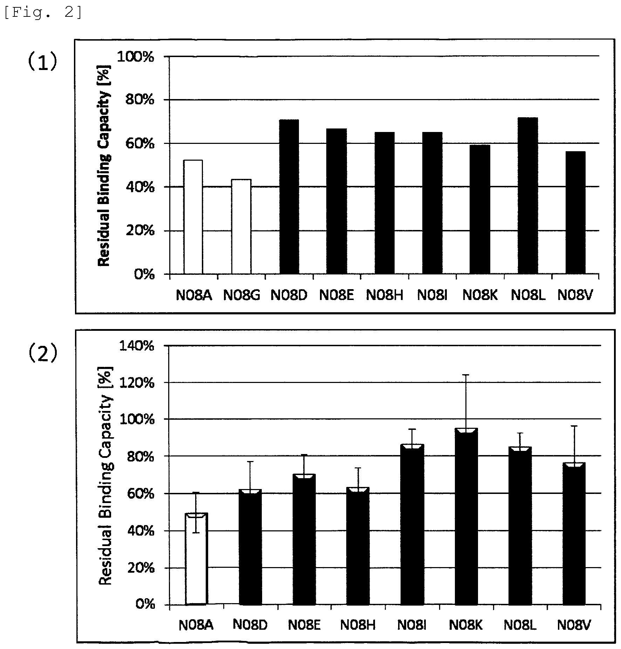

Altered GB1-N08X after the alkali treatment was also diluted to 10 times by using the running buffer to adjust the concentration to 200 nM. The solution was similarly added to the sensor chip at a flow rate of 10 µL/min for 2 minutes to measure an Fc-binding response 1 minute after the addition. The analyte concentration corresponding to the binding response after the alkali treatment was calculated from the previously measured binding response value of 200 nM before the alkali treatment and the slope obtained by the plot of Figure 1. The concentration was regarded as an altered peptide concentration which maintained a binding activity, and the concentration ratio to that before the treatment as 100% was calculated as a residual binding activity. The value is shown in the graph of Figure 2(1).

GB1-N08A described in Non-patent document 7 and GB1-N08G described in Non-patent document 8 were used as Comparative examples to be compared. The mutation position of the present invention is defined as the 7^{th} position in Non-patent document 7. The single residue mutation of GB1-N08G is the most effective in Non-patent document 8. The inventor could find a plurality of mutations of which alkali resistance is significantly superior to the above Comparative examples. The reason why the alkali resistance of GB1-N08G was not good is unclear, but the result of this experiment is considered to be reasonable, since the mutation to Gly basically destabilizes a main chain structure.

Then, a similar evaluation was carried out with N = 4. The result is shown in Figure 2(2). There was variability among numerical values but it is natural that such a degree of variation occurs, since a non-specific damage due to an alkali was evaluated and this evaluation system was sensitive to the damage. In addition, it was repeatable that there were clear significant differences between average values. The result is, therefore, considered to have high reliability. Furthermore, the units of concentrations of a ligand such as SpG and a target to be purified, such as immunoglobulin, are approximately mM in a usual affinity purification, but the unit in this evaluation system was µM or less. This system, therefore, was carried out in a severer condition. It is remarkable result that all of the altered peptides exhibited improved alkali resistance by 10% or more in comparison with GB1-N08A in such an evaluation system.

### (5) Residual Fab-binding activity of GB1#-N08K after alkali treatment

A residual binding activity of GB1#-N08K to Fab was evaluated in a similar condition to Example 2(2) to (4). As a result, the residual binding activity of GB1#-N08A was 68% (N = 4) and the residual binding activity of GB1#-N08K was 80% (N = 4). It was confirmed from the result that even when a base SpG sequence is different, a similar effect can be expected.

### Comparative example

Expression plasmids of GB1-N08A, GB1-N08G and GB1#-N08A used as Comparative examples to be compared in Examples were prepared by a similar method to Example 1(1) by using oligo DNAs f31 to f33 described in Table 2. In addition, protein samples were prepared by a similar method to Example 1(2), and an alkali resistance thereof was evaluated by a similar method to Example 2(2) similarly to the altered peptide according to the present invention.

**Table 2**

| Altered peptide | Oligo DNA | | | Encoding DNA | Amino acid |
|---|---|---|---|---|---|
| | f31 | f32 | f33 | | |
| GB1-N08A.1d | 33 | 6 | 7 | 36 | 39 |
| GB1-N08G.1d | 34 | 6 | 7 | 37 | 40 |
| GB1#-N08A.1d | 35 | 30 | 7 | 38 | 41 |

## Claims

1. An altered immunoglobulin-binding peptide selected from the following (1) to (3):
(1) an immunoglobulin-binding peptide comprising an amino acid sequence of any one of SEQ ID NOs: 1 to 5 wherein an amino acid residue at the 8^{th} position is substituted by Asp, Glu, His, Ile, Lys, Leu or Val;
(2) an immunoglobulin-binding peptide comprising an amino acid sequence specified in the (1) further having deletion, substitution and/or addition of one or more amino acid residues in a region except for the 8^{th} position;
(3) an immunoglobulin-binding peptide comprising an amino acid sequence having a sequence identity of 80% or more with the amino acid sequence specified in the (1), provided that the amino acid substitution mutation specified in the (1) at the 8^{th} position is not further mutated in (3).

2. The altered immunoglobulin-binding peptide according to claim 1, wherein the 8^{th} position of the amino acid sequence specified in the (1) is substituted by Ile, Leu or Lys.

3. The altered immunoglobulin-binding peptide according to claim 1 or 2, wherein an amino acid position of the deletion, substitution and/or addition is one or more positions selected from the group essentially consisting of the 6^{th} position, the 7^{th} position, the 13^{th} position, the 15^{th} position, the 18^{th} position, the 19^{th} position, the 21^{st} position, the 24^{th} position, the 28^{th} position, the 29^{th} position, the 30^{th} position, the 31^{st} position, the 33^{rd} position, the 35^{th} position, the 39^{th} position, the 40^{th} position, the 42^{nd} position and the 47^{th} position in the amino acid sequence specified in the (2).

4. The altered immunoglobulin-binding peptide according to any one of claims 1 to 3, wherein an amino acid position of the deletion, substitution and/or addition is N-terminal and/or C-terminal in the amino acid sequence specified in the (2).

5. An altered immunoglobulin-binding peptide multimer comprising two or more of the altered immunoglobulin-binding peptides according to any one of claims 1 to 4 as domains connected one another.

6. An affinity separation matrix comprising the altered immunoglobulin-binding peptide according to any one of claims 1 to 4 or the altered immunoglobulin-binding peptide multimer according to claim 5 and a water-insoluble carrier, wherein the altered immunoglobulin-binding peptide or the altered immunoglobulin-binding peptide is immobilized as a ligand on the water-insoluble carrier.

7. A method for producing a protein comprising an Fc region and/or a Fab region of an immunoglobulin, comprising:
contacting a liquid sample comprising the protein with the affinity separation matrix according to claim 6; and
separating the protein adsorbed on the affinity separation matrix from the affinity separation matrix.

8. A DNA encoding the altered immunoglobulin-binding peptide according to any one of claims 1 to 4 or the altered immunoglobulin-binding peptide multimer according to claim 5.

9. A vector comprising the DNA according to claim 8.

10. A transformant transformed by the vector according to claim 9.


SEQUENCE LISTING

<110>  Kaneka Corporation

<120>  Stability Improved Immunoglobulin Binding Peptide

<130>  B170199

<150>  JP 2017-071908
<151>  2017-03-31

<160>  41

<170>  PatentIn version 3.5

<210>  1
<211>  56
<212>  PRT
<213>  Streptococcus sp. GX7809

<400>  1

Thr Thr Tyr Lys Leu Ile Leu Asn Gly Lys Thr Leu Lys Gly Glu Thr
1               5                   10                  15

Thr Thr Glu Ala Val Asp Ala Ala Thr Ala Glu Lys Val Phe Lys Gln
            20                  25                  30

Tyr Ala Asn Asp Asn Gly Val Asp Gly Glu Trp Thr Tyr Asp Asp Ala
        35                  40                  45

Thr Lys Thr Phe Thr Val Thr Glu
    50                  55

<210>  2
<211>  56
<212>  PRT
<213>  Streptococcus sp. GX7809

<400>  2

Thr Thr Tyr Lys Leu Val Ile Asn Gly Lys Thr Leu Lys Gly Glu Thr
1               5                   10                  15

Thr Thr Lys Ala Val Asp Ala Glu Thr Ala Glu Lys Ala Phe Lys Gln
            20                  25                  30

Tyr Ala Asn Asp Asn Gly Val Asp Gly Val Trp Thr Tyr Asp Asp Ala
        35                  40                  45

Thr Lys Thr Phe Thr Val Thr Glu
    50                  55

<210>  3
<211>  56

<212>   PRT
<213>   SpG Mutant

<400>   3

Thr Thr Tyr Lys Leu Val Ile Asn Gly Lys Thr Leu Lys Gly Glu Thr
1               5                   10                  15

Thr Thr Lys Ala Val Asp Ala Glu Thr Ala Glu Arg Ala Phe Arg Gln
            20                  25                  30

Tyr Ala Thr Asp Asn Gly Val Glu Gly Met Trp Thr Tyr Asp Pro Ala
        35                  40                  45

Thr Lys Thr Phe Thr Val Thr Glu
    50                  55


<210>   4
<211>   56
<212>   PRT
<213>   SpG Mutant

<400>   4

Thr Thr Tyr Lys Leu Ile Leu Asn Gly Lys Thr Leu Thr Gly Tyr Thr
1               5                   10                  15

Thr Thr Ile Ala Val Asp Ala Ala Thr Ala Glu Lys Val Leu Lys Gln
            20                  25                  30

Phe Ala Asn Asp Asn Gly Val Asp Gly Glu Trp Thr Tyr Asp Asp Ala
        35                  40                  45

Thr Lys Thr Phe Thr Val Thr Glu
    50                  55


<210>   5
<211>   56
<212>   PRT
<213>   SpG Mutant

<400>   5

Thr Thr Tyr Lys Leu Ile Leu Asn Gly Lys Thr Leu Thr Gly Tyr Thr
1               5                   10                  15

Thr Ala Ile Ala Ala Asp Ala Ala Thr Ala Glu Ile Val Leu Lys Gln
            20                  25                  30

Phe Ala Asn Asp Asn Gly Ile Asp Gly Glu Trp Thr Tyr Asp Asp Ala
        35                  40                  45

Thr Lys Thr Phe Thr Val Thr Glu
    50                  55

<210>  6
<211>  69
<212>  DNA
<213>  oligo DNA

<400>  6
accgaagccg ttgatgctgc tacggccgag aaagtgttca aacagtacgc taacgataat        60

ggcgtcgac                                                                69

<210>  7
<211>  69
<212>  DNA
<213>  oligo DNA

<400>  7
cgggaattct tattcggtaa ccgtgaaggt tttggtagcg tcgtcgtagg tccattcacc        60

gtcgacgcc                                                                69

<210>  8
<211>  69
<212>  DNA
<213>  oligo DNA

<400>  8
ctgggatcca ccacctacaa actgatcctg gacggtaaga ccctgaaagg cgagactacg        60

accgaagcc                                                                69

<210>  9
<211>  69
<212>  DNA
<213>  oligo DNA

<400>  9
ctgggatcca ccacctacaa actgatcctg gaaggtaaga ccctgaaagg cgagactacg        60

accgaagcc                                                                69

<210>  10
<211>  69
<212>  DNA
<213>  oligo DNA

<400>  10
ctgggatcca ccacctacaa actgatcctg cacggtaaga ccctgaaagg cgagactacg        60

accgaagcc                                                                69

<210>  11
<211>  69
<212>  DNA
<213>  oligo DNA

<400>  11
ctgggatcca ccacctacaa actgatcctg atcggtaaga ccctgaaagg cgagactacg      60

accgaagcc                                                              69


<210>  12
<211>  69
<212>  DNA
<213>  oligo DNA

<400>  12
ctgggatcca ccacctacaa actgatcctg aaaggtaaga ccctgaaagg cgagactacg      60

accgaagcc                                                              69


<210>  13
<211>  69
<212>  DNA
<213>  oligo DNA

<400>  13
ctgggatcca ccacctacaa actgatcctg ctcggtaaga ccctgaaagg cgagactacg      60

accgaagcc                                                              69


<210>  14
<211>  69
<212>  DNA
<213>  oligo DNA

<400>  14
ctgggatcca ccacctacaa actgatcctg gtcggtaaga ccctgaaagg cgagactacg      60

accgaagcc                                                              69


<210>  15
<211>  171
<212>  DNA
<213>  DNA code

<400>  15
accacctaca aactgatcct ggacggtaag accctgaaag gcgagactac gaccgaagcc      60

gttgatgctg ctacggccga gaaagtgttc aaacagtacg ctaacgataa tggcgtcgac     120

ggtgaatgga cctacgacga cgctaccaaa accttcacgg ttaccgaata a              171


<210>  16
<211>  171
<212>  DNA
<213>  DNA code

<400>  16
accacctaca aactgatcct ggaaggtaag accctgaaag gcgagactac gaccgaagcc      60

gttgatgctg ctacggccga gaaagtgttc aaacagtacg ctaacgataa tggcgtcgac     120

ggtgaatgga cctacgacga cgctaccaaa accttcacgg ttaccgaata a 171

<210> 17
<211> 171
<212> DNA
<213> DNA code

<400> 17
accacctaca aactgatcct gcacggtaag accctgaaag gcgagactac gaccgaagcc 60

gttgatgctg ctacggccga gaaagtgttc aaacagtacg ctaacgataa tggcgtcgac 120

ggtgaatgga cctacgacga cgctaccaaa accttcacgg ttaccgaata a 171

<210> 18
<211> 171
<212> DNA
<213> DNA code

<400> 18
accacctaca aactgatcct gatcggtaag accctgaaag gcgagactac gaccgaagcc 60

gttgatgctg ctacggccga gaaagtgttc aaacagtacg ctaacgataa tggcgtcgac 120

ggtgaatgga cctacgacga cgctaccaaa accttcacgg ttaccgaata a 171

<210> 19
<211> 171
<212> DNA
<213> DNA code

<400> 19
accacctaca aactgatcct gaaaggtaag accctgaaag gcgagactac gaccgaagcc 60

gttgatgctg ctacggccga gaaagtgttc aaacagtacg ctaacgataa tggcgtcgac 120

ggtgaatgga cctacgacga cgctaccaaa accttcacgg ttaccgaata a 171

<210> 20
<211> 171
<212> DNA
<213> DNA code

<400> 20
accacctaca aactgatcct gctcggtaag accctgaaag gcgagactac gaccgaagcc 60

gttgatgctg ctacggccga gaaagtgttc aaacagtacg ctaacgataa tggcgtcgac 120

ggtgaatgga cctacgacga cgctaccaaa accttcacgg ttaccgaata a 171

<210> 21
<211> 171
<212> DNA
<213> DNA code

<400> 21
accacctaca aactgatcct ggtcggtaag accctgaaag gcgagactac gaccgaagcc 60

gttgatgctg ctacggccga gaaagtgttc aaacagtacg ctaacgataa tggcgtcgac 120

ggtgaatgga cctacgacga cgctaccaaa accttcacgg ttaccgaata a 171

<210> 22
<211> 56
<212> PRT
<213> SpG Mutant

<400> 22

Thr Thr Tyr Lys Leu Ile Leu Asp Gly Lys Thr Leu Lys Gly Glu Thr
1               5                   10                  15

Thr Thr Glu Ala Val Asp Ala Ala Thr Ala Glu Lys Val Phe Lys Gln
            20                  25                  30

Tyr Ala Asn Asp Asn Gly Val Asp Gly Glu Trp Thr Tyr Asp Asp Ala
        35                  40                  45

Thr Lys Thr Phe Thr Val Thr Glu
    50                  55

<210> 23
<211> 56
<212> PRT
<213> SpG Mutant

<400> 23

Thr Thr Tyr Lys Leu Ile Leu Glu Gly Lys Thr Leu Lys Gly Glu Thr
1               5                   10                  15

Thr Thr Glu Ala Val Asp Ala Ala Thr Ala Glu Lys Val Phe Lys Gln
            20                  25                  30

Tyr Ala Asn Asp Asn Gly Val Asp Gly Glu Trp Thr Tyr Asp Asp Ala
        35                  40                  45

Thr Lys Thr Phe Thr Val Thr Glu
    50                  55

<210> 24
<211> 56
<212> PRT
<213> SpG Mutant

<400> 24

Thr Thr Tyr Lys Leu Ile Leu His Gly Lys Thr Leu Lys Gly Glu Thr
1               5                   10                  15

Thr Thr Glu Ala Val Asp Ala Ala Thr Ala Glu Lys Val Phe Lys Gln

20                  25                  30

Tyr Ala Asn Asp Asn Gly Val Asp Gly Glu Trp Thr Tyr Asp Asp Ala
        35                  40                  45

Thr Lys Thr Phe Thr Val Thr Glu
    50                  55

<210>  25
<211>  56
<212>  PRT
<213>  SpG Mutant

<400>  25

Thr Thr Tyr Lys Leu Ile Leu Ile Gly Lys Thr Leu Lys Gly Glu Thr
1               5                   10                  15

Thr Thr Glu Ala Val Asp Ala Ala Thr Ala Glu Lys Val Phe Lys Gln
            20                  25                  30

Tyr Ala Asn Asp Asn Gly Val Asp Gly Glu Trp Thr Tyr Asp Asp Ala
        35                  40                  45

Thr Lys Thr Phe Thr Val Thr Glu
    50                  55

<210>  26
<211>  56
<212>  PRT
<213>  SpG Mutant

<400>  26

Thr Thr Tyr Lys Leu Ile Leu Lys Gly Lys Thr Leu Lys Gly Glu Thr
1               5                   10                  15

Thr Thr Glu Ala Val Asp Ala Ala Thr Ala Glu Lys Val Phe Lys Gln
            20                  25                  30

Tyr Ala Asn Asp Asn Gly Val Asp Gly Glu Trp Thr Tyr Asp Asp Ala
        35                  40                  45

Thr Lys Thr Phe Thr Val Thr Glu
    50                  55

<210>  27
<211>  56
<212>  PRT
<213>  SpG Mutant

<400>  27

Thr Thr Tyr Lys Leu Ile Leu Leu Gly Lys Thr Leu Lys Gly Glu Thr
1               5                   10                  15

Thr Thr Glu Ala Val Asp Ala Ala Thr Ala Glu Lys Val Phe Lys Gln
            20                  25                  30

Tyr Ala Asn Asp Asn Gly Val Asp Gly Glu Trp Thr Tyr Asp Asp Ala
        35                  40                  45

Thr Lys Thr Phe Thr Val Thr Glu
    50                  55

<210>  28
<211>  56
<212>  PRT
<213>  SpG Mutant

<400>  28

Thr Thr Tyr Lys Leu Ile Leu Val Gly Lys Thr Leu Lys Gly Glu Thr
1               5                   10                  15

Thr Thr Glu Ala Val Asp Ala Ala Thr Ala Glu Lys Val Phe Lys Gln
            20                  25                  30

Tyr Ala Asn Asp Asn Gly Val Asp Gly Glu Trp Thr Tyr Asp Asp Ala
        35                  40                  45

Thr Lys Thr Phe Thr Val Thr Glu
    50                  55

<210>  29
<211>  69
<212>  DNA
<213>  oligo DNA

<400>  29
ctgggatcca ccacctacaa actgatcctg aaaggtaaga ccctgacagg ctatactacg        60

accatcgcc                                                                69

<210>  30
<211>  69
<212>  DNA
<213>  oligo DNA

<400>  30
accatcgccg ttgatgctgc tacggccgag aaagtgctca aacagttcgc taacgataat        60

ggcgtcgac                                                                69

<210>  31

<211>  171
<212>  DNA
<213>  DNA code

<400>  31
accacctaca aactgatcct gaaaggtaag accctgacag gctatactac gaccatcgcc      60

gttgatgctg ctacggccga gaaagtgctc aaacagttcg ctaacgataa tggcgtcgac     120

ggtgaatgga cctacgacga cgctaccaaa accttcacgg ttaccgaata a              171


<210>  32
<211>  56
<212>  PRT
<213>  SpG Mutant

<400>  32

Thr Thr Tyr Lys Leu Ile Leu Lys Gly Lys Thr Leu Thr Gly Tyr Thr
1               5                   10                  15


Thr Thr Ile Ala Val Asp Ala Ala Thr Ala Glu Lys Val Leu Lys Gln
            20                  25                  30


Phe Ala Asn Asp Asn Gly Val Asp Gly Glu Trp Thr Tyr Asp Asp Ala
        35                  40                  45


Thr Lys Thr Phe Thr Val Thr Glu
    50                  55


<210>  33
<211>  69
<212>  DNA
<213>  oligo DNA

<400>  33
ctgggatcca ccacctacaa actgatcctg gccggtaaga ccctgaaagg cgagactacg      60

accgaagcc                                                              69


<210>  34
<211>  69
<212>  DNA
<213>  oligo DNA

<400>  34
ctgggatcca ccacctacaa actgatcctg ggcggtaaga ccctgaaagg cgagactacg      60

accgaagcc                                                              69


<210>  35
<211>  69
<212>  DNA
<213>  oligo DNA

<400>  35

ctgggatcca ccacctacaa actgatcctg gccggtaaga ccctgacagg ctatactacg      60

accatcgcc                                                              69


<210>  36
<211>  171
<212>  DNA
<213>  DNA code

<400>  36
accacctaca aactgatcct ggccggtaag accctgaaag gcgagactac gaccgaagcc      60

gttgatgctg ctacggccga gaaagtgttc aaacagtacg ctaacgataa tggcgtcgac     120

ggtgaatgga cctacgacga cgctaccaaa accttcacgg ttaccgaata a              171


<210>  37
<211>  171
<212>  DNA
<213>  DNA code

<400>  37
accacctaca aactgatcct gggcggtaag accctgaaag gcgagactac gaccgaagcc      60

gttgatgctg ctacggccga gaaagtgttc aaacagtacg ctaacgataa tggcgtcgac     120

ggtgaatgga cctacgacga cgctaccaaa accttcacgg ttaccgaata a              171


<210>  38
<211>  171
<212>  DNA
<213>  DNA code

<400>  38
accacctaca aactgatcct ggccggtaag accctgacag gctatactac gaccatcgcc      60

gttgatgctg ctacggccga gaaagtgctc aaacagttcg ctaacgataa tggcgtcgac     120

ggtgaatgga cctacgacga cgctaccaaa accttcacgg ttaccgaata a              171


<210>  39
<211>  56
<212>  PRT
<213>  SpG Mutant

<400>  39

Thr Thr Tyr Lys Leu Ile Leu Ala Gly Lys Thr Leu Lys Gly Glu Thr
1               5                   10                  15


Thr Thr Glu Ala Val Asp Ala Ala Thr Ala Glu Lys Val Phe Lys Gln
            20                  25                  30


Tyr Ala Asn Asp Asn Gly Val Asp Gly Glu Trp Thr Tyr Asp Asp Ala
        35                  40                  45

Thr Lys Thr Phe Thr Val Thr Glu
    50                  55

<210>  40
<211>  56
<212>  PRT
<213>  SpG Mutant

<400>  40

Thr Thr Tyr Lys Leu Ile Leu Gly Gly Lys Thr Leu Lys Gly Glu Thr
1               5                   10                  15

Thr Thr Glu Ala Val Asp Ala Ala Thr Ala Glu Lys Val Phe Lys Gln
            20                  25                  30

Tyr Ala Asn Asp Asn Gly Val Asp Gly Glu Trp Thr Tyr Asp Asp Ala
        35                  40                  45

Thr Lys Thr Phe Thr Val Thr Glu
    50                  55

<210>  41
<211>  56
<212>  PRT
<213>  SpG Mutant

<400>  41

Thr Thr Tyr Lys Leu Ile Leu Ala Gly Lys Thr Leu Thr Gly Tyr Thr
1               5                   10                  15

Thr Thr Ile Ala Val Asp Ala Ala Thr Ala Glu Lys Val Leu Lys Gln
            20                  25                  30

Phe Ala Asn Asp Asn Gly Val Asp Gly Glu Trp Thr Tyr Asp Asp Ala
        35                  40                  45

Thr Lys Thr Phe Thr Val Thr Glu
    50                  55